# EUROPEAN PATENT SPECIFICATION

(11) **EP 0 637 956 B1**
(45) Date of publication and mention of the grant of the patent: **30.06.1999**
(21) Application number: 93911903.8
(22) Date of filing: 30.04.1993
(51) Int. Cl.: A61K 31/165, A61K 31/195

(54) **USE OF REMACEMIDE FOR THE TREATMENT OF PARKINSON'S DISEASE**
VERWENDUNG VON REMACEMID ZUR BEHANDLUNG DES MORBUS PARKINSON
UTILISATIION DE REMACEMIDE DANS LE TRAITEMENT DE LA MALADIE DE PARKINSON

(30) Priority: 02.05.1992 GB 9209599
(43) Date of publication of application: 15.02.1995
(73) Proprietor: Astra Aktiebolag, 151 85 Södertälje (SE)
(72) Inventor: JOHNSON, Peter, Chasewood House, Aspley Guise Bedfordshire MK17 8ER (GB); WATKINS, Barry, Ernest, Fairport, NY 14450 (US); HARRIS, Eric, William, Rochester, NY 14620 (US)
(74) Representative: Summersell, Richard John, Dr.
(86) International application number: GB9300908
(87) International publication number: WO9321910

(56) References cited:
- EP-A- 0 427 427
- EP-A- 0 434 173
- ANNALS OF NEUROLOGY vol. 28, no. 4, 1990, pages 539 - 546 KLOCKGETHER, T. ET AL 'NMDA ANTAGONISTS POTENTIATE ANTIPARKINSONIAN ACTIONS OF L-DOPA IN MONOAMINE DEPLETED RATS' cited in the application
- ABSTRACTS OF PAPERS OF THE AMERICAN CHEMICAL SOCIETY vol. 201, no. 1-2, 1991, page 22 GRIFFITH, R. ET AL 'PRECLINICAL NEUROPROTECTIVE PROFILE OF REMACEMIDE, A PRODRUG NMDA ANTAGONIST'
- SOC. NEUROSCI. ABSTR. vol. 18, no. 1-2, 1992, page 1080 ELLER, R.V. ET AL 'REMACEMIDE AND L DOPA SYNERGIZE TO REVERSE AKINESIA IN MONOAMINE-DEPLETED RATS'

## Description

This invention relates to a novel method of treatment of patients suffering from Parkinson's disease, the novel use of a known compound in the treatment of Parkinson's disease, and to pharmaceutical products and formulations for use in that treatment.

Parkinson's disease is thought to be caused by arteriosclerotic changes in the basal ganglia and is characterized by rhythmical muscular tremors and rigidity of movement.

European Patent Application 279937 discloses 2-amino-N-(1,2-diphenyl-1-methylethyl)-acetamide (hereinafter referred to as "remacemide") and its pharmaceutically acceptable salts, and their use as anti-epileptic agents. European Patent Application 427427 discloses the use of remacemide and its pharmaceutically acceptable salts in the treatment of neurodegenerative diseases: however, their use in the treatment of Parkinson's disease is not mentioned. Remacemide and its pharmaceutically acceptable salts are thought to be non-competitive N-methyl-D-aspartate (NMDA) antagonists.

3-Hydroxy-L-tyrosine (hereinafter referred to as "L-dopa") is a well known agent for use in the treatment of Parkinson's disease.

The use of NMDA antagonists to potentiate the antiparkinsonian action of L-dopa in monoamine-depleted rats has been disclosed by Klockgether *et al* (Annals of Neurology, vol 28, No 4, p539, 1990).

It has now been found that administration of remacemide, or a pharmaceutically acceptable salt thereof, in conjunction with L-dopa, to a patient suffering from Parkinson's disease is particularly beneficial. Remacemide, or a pharmaceutically acceptable salt thereof, may also be useful in the treatment of Parkinson's disease on its own.

Thus, according to the present invention, there is provided:
the use of remacemide, or a pharmaceutically acceptable salt thereof, as active ingredient in the manufacture of a medicament for the treatment of Parkinson's disease; and
a pharmaceutical product containing remacemide or a pharmaceutically acceptable salt thereof, and L-dopa, as a combined preparation for separate, simultaneous or sequential use in the treatment of Parkinson's disease.

The administration of the active agents (ie remacemide, or a pharmaceutically acceptable salt thereof, and L-dopa) in conjunction with one another is advantageous because, for example, the therapeutic effect of L-dopa is particularly enhanced, permitting the therapeutic effect of L-dopa to be achieved at a dosage level below that normally employed, thus allowing the unwanted side effects of L-dopa (for example neurotoxicity) to be eliminated or substantially mitigated.

Pharmaceutically acceptable salts of remacemide include acid addition salts, for example the hydrochloride and hydrobromide salts.

By "simultaneous use" is meant administration of the two active agents in a single pharmaceutical formulation. Thus, according to a further aspect of the invention, there is provided a pharmaceutical formulation comprising remacemide or a pharmaceutically acceptable salt thereof, L-dopa, and a pharmaceutically acceptable adjuvant, diluent or carrier.

By "sequential use" is meant administration of the two active agents in separate pharmaceutical formulations, but with a short period of time between each administration, for example up to 5 minutes.

By "separate use" is meant administration of the two active agents in separate pharmaceutical formulations, but with a relatively longer period of time between each administration, for example from 5 minutes to 12 hours.

The active agents may be administered by any convenient route, for example parenterally, rectally, and of particular interest - orally. Pharmaceutical formulations comprising the active agents include solid dosage forms, for example tablets, pills, capsules, powders, granules, and suppositories for oral, parenteral or rectal administration; and liquid dosage forms, for example sterile parenteral solutions or suspensions, suitably flavoured syrups, flavoured emulsions with edible oils such as cottonseed oil, sesame oil, coconut oil and peanut oil, and elixirs and similar pharmaceutical vehicles.

Solid compositons may be prepared by mixing the active agents with pharmaceutical carriers, for example conventional tabletting ingredients such as corn starch, lactose, sucrose, sorbitol, talc, stearic acid, magnesium stearate, dicalcium phosphate, gums and other diluents, for example water, to form a homogenous preformulation composition in which the active agents are uniformly dispersed. The solid dosage forms may be coated or otherwise compounded to prolong the action of the composition.

In general, in humans, a total oral daily dose of from about 150-800mg of L-dopa, in conjunction with from 50 to 300mg of remacemide or a pharmaceutically acceptable salt thereof, is suitable. The daily dose of each agent may be divided into, for example, up to 4 smaller amounts to be taken during the course of each day.

Thus, in the pharmaceutical products and formulations according to the invention, the active agents are preferably present or used in a w/w ratio of remacemide or a pharmaceutically acceptable salt thereof to L-dopa of from 1:2-1:4, for example 1:3.

The invention is illustrated by the following Example.

### Example

Male Sprague-Dawley rats (125-150g) were rendered akinetic by administration of reserpine (so as to produce a monoamine depleted Parkinson's disease-like state, 5 mg/kg) 24 hours prior to testing. Motor activity was quantified using cages equipped with infra-red sensor beams. In rats treated with reserpine, L-Dopa increased horizontal locomotor activity in a dose-dependent fashion. When a sub-therapeutic dose of L-dopa (75mg/kg. i.p.) was co-administered with remacemide (5-40mg/kg, p.o.) there was a dose-dependent increase in horizontal locomotor activity.

In a comparison with the NMDA antagonist 10,11-dihydro-5-methyl-5H-dibenzo[a,d]-cyclohepten-5,10-imine (MK-801), remacemide was found to potentiate the effect of L-dopa over a broader dose range.

## Claims

1. The use of remacemide or a pharmaceutically acceptable salt thereof, as active ingredient in the manufacture of a medicament for the treatment of Parkinson's disease.

2. A pharmaceutical product containing remacemide or a pharmaceutically acceptable salt thereof, and L-dopa, as a combined preparation for separate, simultaneous or sequential use in the treatment of Parkinson's disease.

3. A pharmaceutical product as claimed in claim 2, wherein remacemide or a pharmaceutically acceptable salt thereof, and L-dopa, are present in a w/w ration of from 1:2-1:4.

4. A pharmaceutical formulation comprising remacemide or a pharmaceutically acceptable acid addition salt thereof, L-dopa, and a pharmaceutically acceptable adjuvant, diluent or carrier.

5. A pharmaceutical formulation as claimed in claim 4, wherein remacemide or a pharmaceutically acceptable salt thereof, and L-dopa, are present in a w/w ratio of from 1:2-1:4.

## Patentansprüche

1. Verwendung von Remacemid oder einem seiner pharmazeutisch unbedenklichen Salze als Wirkstoff zur Herstellung eines Medikamentes für die Behandlung von Parkinson-Syndrom.

2. Pharmazeutisches Produkt, enthaltend Remacemid oder eines seiner pharmazeutisch unbedenklichen Salze und L-Dopa als Kombinationspräparat für getrennte, gleichzeitige oder aufeinanderfolgende Verwendung in der Behandlung von Parkinson-Syndrom.

3. Pharmazeutisches Produkt nach Anspruch 2, bei dem Remacemid oder eines seiner pharmazeutisch unbedenklichen Salze und L-Dopa in einem Gewichtsverhältnis von 1:2 bis 1:4 vorliegt.

4. Pharmazeutische Formulierung, enthaltend Remacemid oder eines seiner pharmazeutisch unbedenklichen Salze, L-Dopa und einen pharmazeutisch unbedenklichen Zusatzstoff, Verdünnungsmittel oder Trägerstoff.

5. Pharmazeutische Formulierung nach Anspruch 4, bei dem Remacemid oder eines seiner pharmazeutisch unbedenklichen Salze und L-Dopa in einem Gewichtsverhältnis von 1:2 bis 1:4 vorliegen.

## Revendications

1. Utilisation de remacémide ou d'un sel pharmaceutiquement acceptable de celui-ci, en tant qu'ingrédient actif dans la fabrication d'un médicament pour le traitement de la maladie de Parkinson.

2. Produit pharmaceutique contenant du remacémide ou un sel pharmaceutiquement acceptable de celui-ci, et de la L-dopa, sous forme d'une préparation combinée destinée à une utilisation séparée, simultanée ou séquentielle dans le traitement de la maladie de Parkinson.

3. Produit pharmaceutique suivant la revendication 2, dans lequel le remacémide ou un sel pharmaceutiquement acceptable de celui-ci et la L-dopa sont présents dans un rapport p/p allant de 1:2 à 1:4.

4. Composition pharmaceutique comprenant du remacémide ou un sel d'addition acide pharmaceutiquement acceptable de celui-ci, de la L-dopa et un adjuvant, diluant ou support pharmaceutiquement acceptable.

5. Composition pharmaceutique suivant la revendication 4, dans laquelle le remacémide ou un sel pharmaceutiquement acceptable de celui-ci et la L-dopa sont présents dans un rapport p/p allant de 1:2 à 1:4.
